Europäisches Patentamt

European Patent Office

Office européen des brevets

(19)

(11) Numéro de publication: **0 270 439 B1**

(12) # FASCICULE DE BREVET EUROPEEN

(45) Date de publication du fascicule du brevet:
08.08.90

(51) Int. Cl.⁵: **G01L 3/24**, G01L 3/14

(21) Numéro de dépôt: 87402584.4

(22) Date de dépôt: 17.11.87

(54) Procédé et dispositif pour mesurer le couple transmis par la roue motrice d'un cycle.

(30) Priorité: 19.11.86 FR 8616109

(43) Date de publication de la demande:
08.06.88 Bulletin 88/23

(45) Mention de la délivrance du brevet:
08.08.90 Bulletin 90/32

(84) Etats contractants désignés:
BE CH DE ES GB IT LI NL

(56) Documents cités:
DE-A- 3 150 149

PATENT ABSTRACTS OF JAPAN,
vol. 8, no. 177 (M-317)[1614], 15 août 1984; &
JP-A-59 69 541 (ONO SOTSUKI K.K.) 19-04-1984
PROCEEDINGS OF THE IEEE 1982 NATIONAL
AEROSPACE AND ELECTRONICS CONFERENCE,
Dayton, 18-20 Mai 1982, vol. 1, Seiten 194-196, IEEE; D.B.
REYNOLDS et al.: "Digital speedometer and crank
position indicator for the monark bicycle ergometer"

(73) Titulaire: Sté. Look, Rue de la Pique, F-58000 Nevers(FR)

(72) Inventeur: Mercat, Jean-Pierre, 24, rue Gambetta,
F-37110 Château-Renault(FR)

(74) Mandataire: Tony-Durand, Serge, Cabinet
Tony-Durand 77, rue Boissière, F-75116 Paris(FR)

ACTORUM AG

**EP 0 270 439 B1**

**Description**

La présente invention concerne un procédé pour mesurer le couple transmis par la roue motrice d'un cycle, et en particulier la roue motrice d'une bicyclette.

La présente invention concerne également un dispositif pour la mise en œuvre de ce procédé.

On connait d'après le FR-A-2 394 790 un dispositif du genre indiqué au début dans lequel un galet est appliqué sous la pression d'un ressort contre le brin moteur de la chaîne de transmission de la bicyclette. Plus l'effort transmis par la chaîne est important, plus le brin moteur de celle-ci tend à se redresser en comprimant le ressort. Il existe donc une règle de correspondance entre la flèche du ressort et l'effort transmis par le brin moteur de la chaîne.

Ce dispositif présente de multiples inconvénients. Il est lourd. Ses indications sont imprécises compte tenu des parasites correspondant aux vibrations de fonctionnement de la chaîne. L'effort mesuré est l'effort transmis par la chaîne qui, à valeur égale, peut correspondre à des couples différents sur la roue motrice si la bicyclette est équipé d'un dispositif de changement de vitesse. De plus, le dispositif connu introduit un frottement sur la chaîne.

Le DE 31 50 149 décrit une bicyclette équipée d'un dispositif de mesure de couple dont les organes de détection peuvent être placés en différents endroits. Toutefois, à l'exception d'exemples de réalisation qui tous correspondent à la localisation des organes de détection à l'endroit du moyeu de la roue motrice arrière, le texte de ce document se borne à citer la possibilité d'implantation d'un appareil d'enregistrement sur les pédales ou les manivelles du pédalier, ou bien encore sur la chaine de transmission ou les organes associés à celle-ci (plateau avant d'entrainement ou pignon arrière entrainé). Mais le DE 31 50 149 ne fournit aucune information sur les solutions pratiques susceptibles d'être adoptées dans les différents cas ainsi envisagés.

Ce n'est que dans le cas d'une localisation à l'endroit du moyeu de la roue motrice que ce document fournit des suggestions concrètes. Cependant les solutions envisagées sont complexes et coûteuses puisqu'elles impliquent une réalisation particulière du moyeu de façon que celui-ci comporte deux parties distincts, à savoir une partie "menante" et une partie "menée" entre lesquelles peut être interposé un élément élastique de liaison, le système de mesure comprenant des moyens de détection des positions angulaires relatives de l'une et l'autre des deux parties ainsi prévues sur le moyeu. Cependant du fait même du très grand rapprochement de ces deux parties, ces écarts angulaires sont très réduits, ce qui ne permet pas des mesures précises.

C'est pourquoi le but de l'invention est de proposer un procédé et un dispositif dans lesquels la mesure du couple, ou d'une grandeur liée à celui-ci, soit effectuée d'une manière simple et précise sans que cela entraîne une modification de certains des éléments constitutifs du cycle lui-même.

A cet effet l'invention a pour premier objet un procédé pour mesure le couple transmis par la roue motrice d'un cycle, ou une grandeur mécanique fonction dudit couple, consistant à mesurer l'écart angulaire entre une partie menante et une partie menée de l'un des éléments rotatifs du cycle correspondant, faisant partie de ses éléments participant à sa propulsion, caractérisé en ce que l'on mesure la déformation angulaire engendrée dans la roue motrice elle-même lors du roulement du cycle, par mesure de l'écart angulaire existant de ce fait entre une région centrale de cette roue motrice et une région périphérique de cette même roue, et ce en détectant le passage de deux séries distinctes de repères portés respectivement par la région centrale de la roue motrice et une région périphérique de cette même roue, et on détermine le couple, ou la grandeur qui lui est liée, en exploitant une règle de correspondance prédéterminée entre l'écart détecté et le coupe.

Ainsi, c'est la déformation de la roue motrice elle-même, sous l'effet du couple, qui est détectée et transformée en une mesure de ce couple. A ce sujet il convient de noter qu'en présence du DE-A-3 150 149, il n'était pas évident d'utiliser la déformation de la roue motrice elle-même pour la mesure du couple, compte tenu que ce document se bornait à envisager la mesure de l'écart angulaire existant entre deux parties distinctes du moyeu d'une telle roue en adoptant un agencement particulier permettant une certaine liberté de débattement angulaire entre ces deux parties. Une solution de ce genre ne pouvait donc en aucune façon suggérer celle adaptée dans le procédé selon l'invention et qui consiste à détecter la déformation existant entre la partie centrale de la roue et la partie périphérique de celle-ci. Du reste il ne paraissait pas à priori évident que la déformation de la roue motrice soit suffisante pour en déduire, de façon fidèle et précise, la valeur du couple de transmission.

Or la solution matérialisée par le procédé selon l'invention a pour avantage que l'on détecte un écart angulaire entre la région centrale de la roue et sa région périphérique, donc entre deux régions considérablement espacées. Ceci permet donc une mesure précise. Par ailleurs l'implantation des organes de détection ne soulève pas de problème particulier puisque les uns sont situés dans la région centrale de la roue et les autres dans sa région périphérique. D'autre part, comme on se contente d'apprécier une position angulaire relative, on peut le faire avec des moyens légers n'introduisant pas de frottement. De plus, il n'existe pas de vibrations notables entre le moyeu et la jante d'une roue dans le sens circonférentiel, de sorte que la mesure est très peu affectée par les parasites.

Selon un second aspect de l'invention, le dispositif pour la mise en oeuvre du procédé ci-dessus est caractérisé en ce qu'il comprend des premiers moyens détecteurs pour détecter la position angulaire du moyeu de la roue motrice autour de son axe, des second moyens détecteurs pour détecter la position an-

2

gulaire de la jante de la roue motrice autour de son axe, des moyens reliés aux premiers et seconds moyens détecteurs et conçus pour délivrer un signal représentatif de l'écart angulaire entre la jante et le moyeu autour de l'axe de la roue motrice, et des moyens pour indiquer le couple transmis ou la grandeur qui lui est liée par exploitation de la règle de corresondance entre l'écart angulaire et le couple transmis.

D'autres particularités et avantages de l'invention ressortiront encore de la description ci-après.

Aux dessins annexés, donnés à titre d'exemples non limitatifs :

- la figure 1 est une vue en élévation latérale d'une bicyclette selon l'invention,
- la figure 2 est une vue partielle à échelle agrandie de la roue motrice de la bicyclette de la figure 1 et des capteurs avec arrachement,
- la figure 3 est une vue en coupe selon la ligne III-III de la figure 2,
- les figurs 4 et 5 sont des vues des détails IV et V de la figure 3 respectivement, et
- la figure 6 est une vue du schéma bloc du circuit d'évaluation prévu sur la bicyclette de la figure 1.

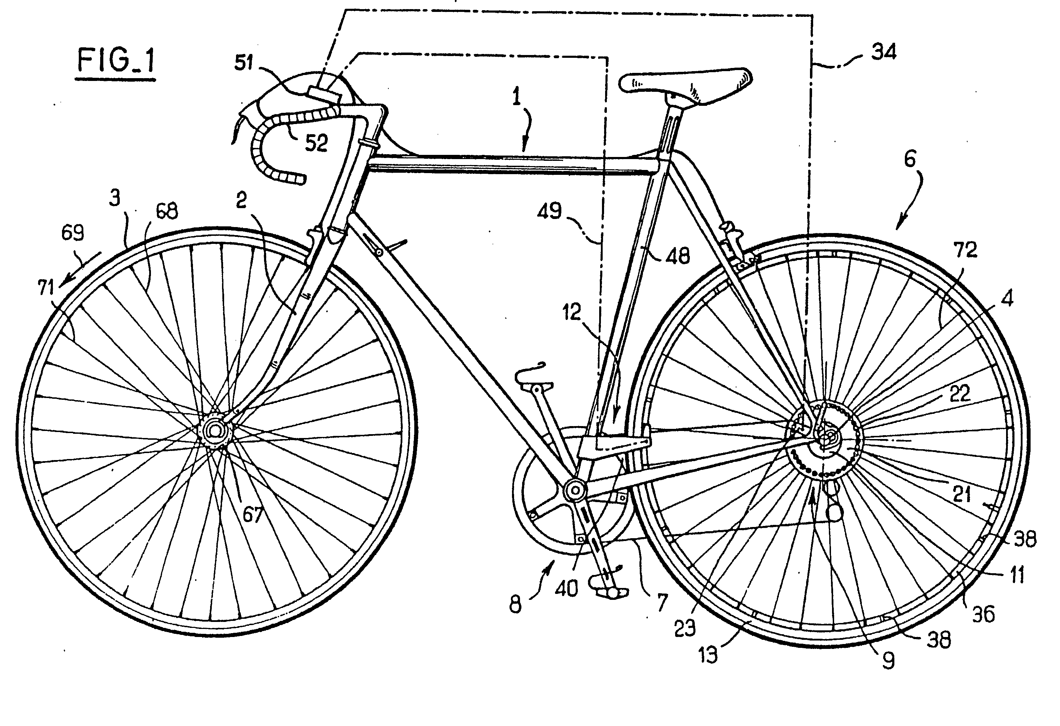

Comme le montre la figure 1, la bicyclette comprend un cadre 1 auquel est articulée une fourche 2 supportant une rotation une roue avant 3. Le cadre 1 supporte lui-même, en rotation autour d'un axe horizontal 4, une roue arrière motrice 6 entraînée en rotation par l'intermédiaire d'une chaîne de transmission 7 à partir d'un pédalier 8.

Conformément à l'invention, la bicyclette comprend des premiers moyens détecteurs 9 pour détecter la position angulaire d'un moyeu 11 de la roue 6 autour de son axe 4, et des second moyens détecteurs 12 pour détecter la position angulaire d'une jante 13 de la roue 6 autour de son axe.

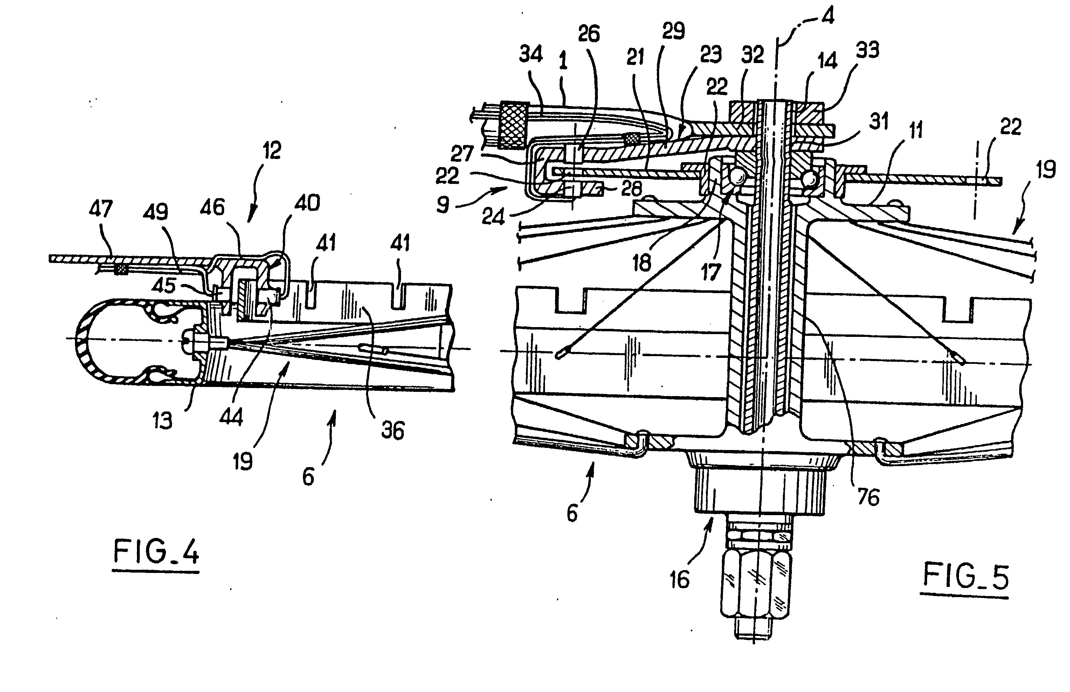

Comme le montre la figure 5, le moyeu 11 est supporté sur un arbre de moyeu 14 au moyen d'un roulement à billes à roue libre 16 du côté destiné à recevoir les pignons de chaîne (non représentés), et par un roulement à billes 17 du côté opposé aux pignons de chaîne. Le roulement 17 est monté entre l'arbre 14 et un collet 18 que présente le moyeu 11 en direction opposée au rayonnage, lequel sera décrit plus en détail ultérieurement.

Les premier moyens détecteurs 9 comprennent un élément - ou disque - 21 présentant une ouverture centrale dans laquelle il reçoit une bague 22 à laquelle il est fixé. La bague 22 est emmanchée à force sur le collet 26.

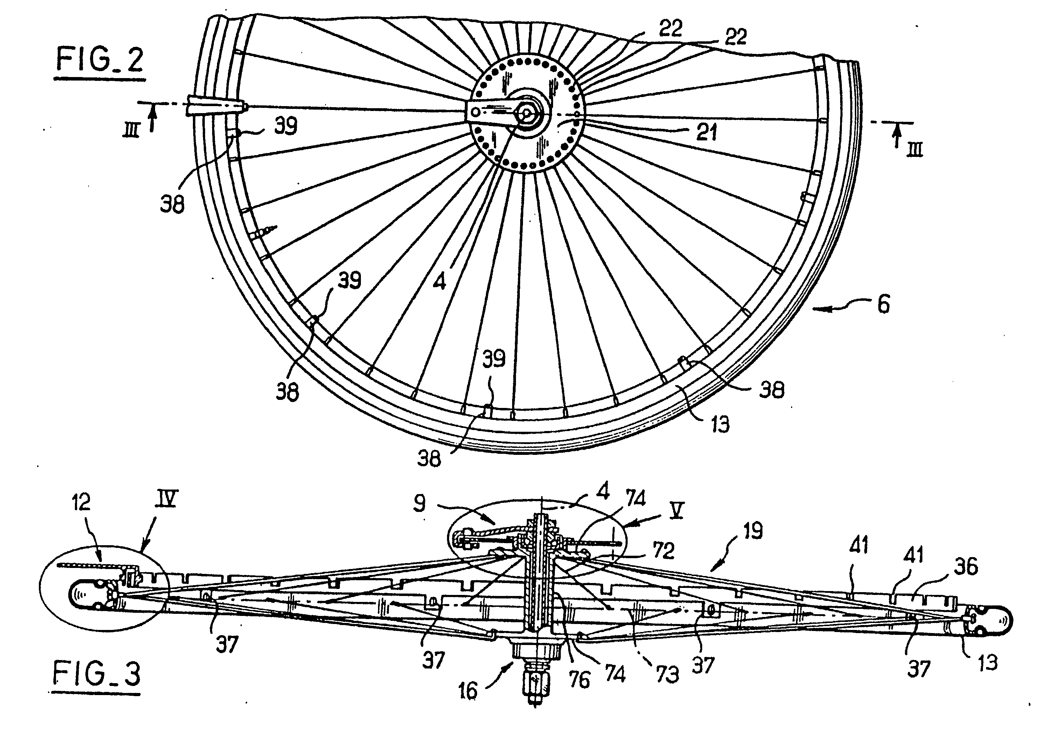

Comme le montre la figure 2, le disque 21 est traversé par une série d'ajours 22 de forme circulaire régulièrement répartis au voisinage du bord périphérique du disque 21 le long d'un cercle centré sur l'axe 4. Les ajours 22 sont constitués par des perçages circulaires tous identiques.

Comme le montre la figure 5, les premiers moyens détecteurs 9 comportent de plus un capteur 23 comprenant une diode émettrice d'infrarouges 24 et une photodiode 26, montées l'une en face de l'autre de part et d'autre du disque 21 de façon que les ajours 22 passent successivement entre elles, lorsque, en service, la roue 6 tourne autour de l'axe 4 et entraîne avec elle le disque 21.

Les diodes 24 et 26 sont réunies rigidement l'une à l'autre par un cavalier 27 qui chevauche le disque 21 et dont les bras 28 et 29 sont dirigés radialement vers l'axe 4. Le bras 28, relativement court, porte la diode 24 entre le disque 21 et le rayonnage 19. L'autre bras 29 porte la diode 26 en regard de la face du disque 21 opposée au rayonnage 19. L'extrémité du bras 29 présente un orifice 31 enfilé sur l'axe 4 et serré entre la bague intérieure du roulement 17 et une oreille terminale 32 du cadre 1, le tout étant bloqué par un écrou 33 appuyant sur la face extérieure de l'oreille 32. Une liaison quadrifilaire 34 fixée au cadre 1 et à l'étrier 27 comprend deux fils d'alimentation de la diode infrarouge 24 et deux fils pour le départ du signal élaboré par la photodiode 26.

Les seconds moyens de détection 12 comprennent un élément - ou cerceau 36 - de forme générale cylindrique ayant pour axe l'axe 4 et dont le diamètre est légèrement inférieur au diamètre intérieur de la jante 13. Le cerceau 36 est situé latéralement contre le rayonnage 19, du même côté que les premiers moyens détecteurs 9. Le long de son bord annulaire adjacent au rayonnage 19, le cerceau 36 présente des languettes 37 appuyées contre la face intérieure de la jante 13 par l'intermédiaire d'entretoises tubulaires 38. Des vis 39, traversant les languettes 37 et les entretoises 38, se vissent dans la jante 13 pour solidariser le cerceau 36 avec la jante 13.

Sur son bord éloigné du rayonnage 19, le cerceau 36 présente des ajours - ou créneaux 41 - (figure 3) tous identiques et régulièrement répartis autour de l'axe 4. De plus, le nombre total des créneaux 41 est égale au nombre total des ajours 22.

Les moyens de détection 12 comportent encore un capteur 40 (figure 4) comprenant une diode émettrice d'infrarouges 44 et une photodiode 45 disposées l'une en face del'autre, l'une à l'intérieur et l'autre à l'extérieur du cerceau 36, de façon que les créneaux 41 défilent successivement entre elles lorsque le cerceau 36 est entrainé en rotation autour de l'axe 4 par la roue 6. Les diodes 44 et 45 sont liées rigidement l'une à l'autre au moyen d'un cavalier 46 chevauchant le cerceau 36 par son bord éloigné du rayonnage 19. Le cavalier 46 est porté par une patte 47 de fixation au montant de selle 48 (figure 1) de la bicyclette. Une liaison quadrifilaire 49 fixée à la patte 47 comprend deux fils d'alimentation électrique de la diode infrarouge 44 et deux fils de départ du signal élaboré par la photodiode 45.

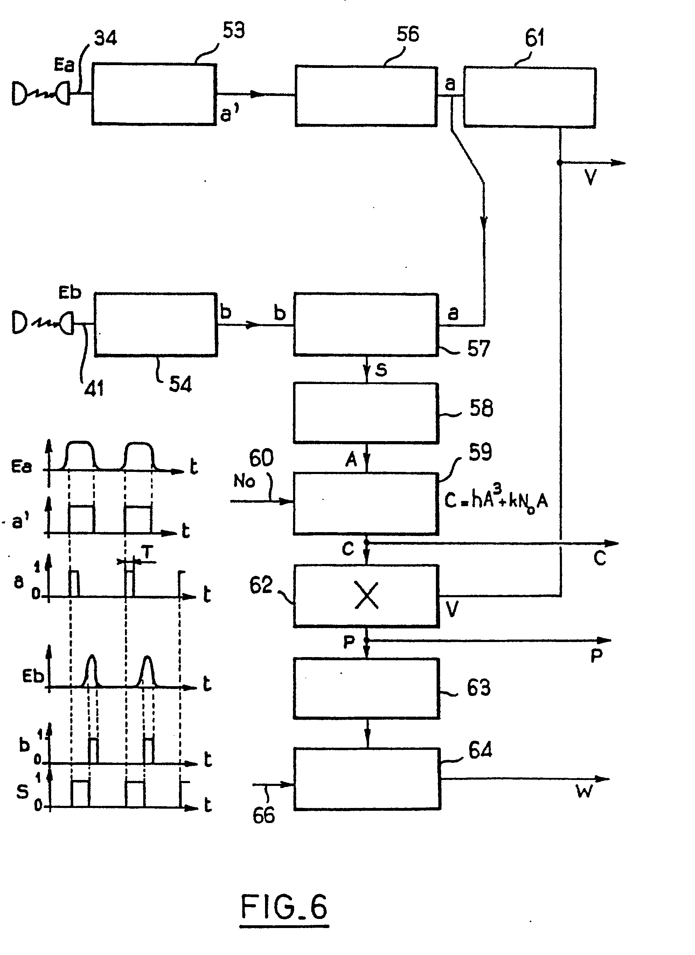

Comme le montre la figure 6, les signaux Ea et Eb, émis respectivement par les photodiodes 26 et 45 sont sensiblement binaires, l'un des niveaux correspondant au cas où un ajour 22, 41 se trouve entre la diode et la photodiode, l'autre niveau correspondant au cas où le métal du disque 21 ou du cerceau 36 se trouve entre la diode et la photodiode.

Chaque signal est amené, par les lignes 34, 49 (représentées schématiquement à la figure 1), à un boîtier électronique 51 fixé au guidon 52 de la bicyclette et contenant au moins une pile électrique pour l'alimentation de l'ensemble du dispositif.

Dans le boîtier 51, les signaux sensiblement binaires précités sont transformés en signaux rectangulaires au moyen d'une bascule 53 et 54 respectivement. De plus, le signal $a'$ émis par la bascule 53 associée à la photodiode 26 est traité dans une bascule monostable 56 fournissant à sa sortie un signal binaire $a$ constitué par un pic rectangulaire de durée T invariable, dont le flan de montée coïncide temporellement avec le flanc de montée des signaux rectangulaires $a'$.

Le signal $a$ élaboré par la bascule monostable 56 et le signal $b$ élaboré par la bascule 54 sont envoyés chacun à l'une respective des entrées d'un déphaseur 57 fournissant à sa sortie un signal binaire rectangulaire S dont chaque alternance a un flanc de montée et un flanc de descente qui, temporellement, coïncident respectivement avec le flanc montant d'un pic du signal $a$ et avec le flanc montant du pic qui, dans le signal $b$, suit immédiatement dans le temps le pic précité du signal $a$.

Ainsi, à chaque période, la durée pendant laquelle le signal S est au niveau 1 correspond au retard du signal $b$ par rapport au signal $a$ ou $a'$, c'est-à-dire au retard du signal $Eb$ par rapport au signal $Ea$, c'est-à-dire encore au retard des créneaux 41 du cerceau 36 relativement aux ajours 22 du disque 21.

Au montage, le cerceau 36 est positionné angulairement autour de l'axe 4 de façon que les signaux $Ea$ et $Eb$ montent en même temps lorsque la roue n'est soumis à aucun cisaillement en direction circonférentielle, c'est-à-dire lorsqu'aucun couple n'est transmis. Dès lors, la durée pendant laquelle le signal S au niveau 1 dans chaque période est indicative de l'écart angulaire relatif existant entre la jante et le moyeu de la roue par rapport à la position de référence correspondant à un couple transmis égal à 0.

Le signal S est délivré à l'entrée d'un intégrateur 58 dont la sortie analogique A est proportionnelle, à chaque instant, à la durée pendant laquelle le signal S est au niveau A au cours de chaque période. Le signal A est donc représentatif de l'angle de décalage qui existe entre la jante et le moyeu de la roue 6 par rapport à la position de référence précitée.

Le signal A est fourni à l'entrée d'un calculateur 59, lequel élabore un signal analogique C représentatif du couple transmis par la roue 6, en exploitant une règle de correspondance entre ledit couple et l'écart angulaire A. Dans l'exemple, cette règle donne le couple C en fonction de la variable A au moyen de la fonction du troisième degré suivante :

$$C = hA_3 + kN)A$$

dans laquelle $N_0$ est la tension à vide des rayons du rayonnage 19.

On verra plus loin comment sont déterminés h et k, et plus généralement comment réaliser une roue satisfaisant à cette règle de correspondance.

La sortie C du calculateur 59 est directement utilisable pour faire fonctionner un dispositif d'affichage de la valeur du couple transmis par la roue motrice 6.

De plus, la sortie $a$ de la bascule monostable 56 est fournie à l'entrée d'un intégrateur 61 dont la sortie analogique V est proportionnelle à la vitesse instantanée de rotation de la roue 6 autour de son axe 4, et par conséquence à la vitesse instantanée de déplacement de la bicyclette. Ce signal est obtenu en élaborant, à chaque instant l'intégrale temporelle du signal $a$ depuis un instant précédent d'une durée prédéterminée invariable l'instant ou le résultat est fourni.

Le signal C d'une part et le signal V, proportionnel à la vitesse de rotation de la roue 6, d'autre part sont envoyés chacun à l'une des entrées d'un multiplicateur analogique 62 dont la sortie analogique P est représentative de la puissance transmise par la roue motrice 6. (On rappelle que la puissance est égale au produit du couple multiplié par la vitesse de rotation). Le signal P est directement utilisable pour commander l'affichage de la puissance développée par le cycliste au niveau de la roue arrière.

Le signal V est lui-même directement utilisable pour commander un affichage de la vitesse de déplacement de la bicyclette.

Le signal P est d'autre part fourni à l'entrée d'un convertisseur tension fréquence 63 dont le signal de sortie est fourni à l'entrée d'un compteur 64 qui, en totalisant le nombre d'impulsions qu'il reçoit à partir d'un certain instant auquel une remise à zéro a été effectuée au moyen d'une commande spécifique 66, fournit sur sa sortie un signal W permettant de commander l'affichage d'un totalisateur d'énergie transmise par la roue 6.

En ce qui concerne le mode de calcul de cette énergie, ou rappelle que l'énergie produit pendant un temps élémentaire $dt$ est égale au produit de la puissance P par le temps élémentaire $dt$.

Comme le montre la figure 1, la roue avant 3 de la bicyclette est d'un type classique à rayonnage croisé, c'est-à-dire que les rayons, au lieu d'apparaître dirigés radialement lorsque la roue est vue axialement, sont inclinés, notamment par rapport aux plans axiaux passant par leurs extrémités. A partir du moyeu 67 de la roue 3, certains rayons 68 sont inclinés vers l'avant relativement au sens 69 de rotation de la roue 3, et d'autres rayons 71, croisant les rayons 68, sont inclinés vers l'arrière relativement au sens 69.

Le rayonnage 19 de la roue arrière motrice 6 est d'un type spécial dans lequel les rayons 72 apparaissent dirigés radialement lorsque la roue 6 est vue axialement. En réalité, leur direction n'est pas tout à fait radiale, comme le montre la figure 3. Plus particulièrement, les extrémités radialement extérieures des rayons 72 sont toutes fixées à la jante 13 le long d'un cercle idéal commun 73. Les extrémités radialement extérieures des rayons 72 sont régulièrement réparties le long du cercle 73. A partir de ce cercle, les rayons se répartissent en deux nappes coniques opposées, disposées symétriquement de part et d'autre du plan moyen de la roue, passant par le cercle 73. A leur extrémité radialement intérieure, les rayons 72 sont fixés à l'un ou à l'autre de deux flasques 74 que présente le moyeu 11 aux deux extrémités d'un tube central 76 entourant l'axe 14 entre les roulements 16 et 17. Le long de la jante 13, il y a alternance de rayons 72 appartenant à l'une et à l'autre nappe.

Il est à noter que la roue 6 ainsi rayonnée est réalisable à partir d'une jante, d'un moyeu et de rayons qui conviendraient pour réaliser une roue 3, la seule différence de montage consistant en ce que les rayons se succèdent dans le même ordre à leur extrémité radialement intérieure et à leur extrémité radialement extérieure.

Une telle roue satisfait à la relation suivante, qui se démontre mathématiquement et se vérifie expérimentatlement avec une erreur de quelques % seulement :

$C = hA3 + kN)A(1)$

dans laquelle :

C est le couple en N.m

A est l'écart angulaire en degrés entre la jante et le moyeu de la roue par rapport à la position de référence ;

$N_0$ est la tension à vide des rayons exprimée en N

$$h = \frac{n \pi^3 R^2 r^2 \cdot ES}{11\ 664.10^3\ (R - r)^3}$$

$$k = \frac{n \pi Rr}{180\ (R - r).}$$

Dans ces dernières expressions :

- n est le nombre de rayons
- R est le rayon de la jante, ou plus exactement la distance entre le point d'attache radialement extérieur des rayons 72 et l'axe de la roue
- r est la distance entre le point d'attache radialement intérieur des rayons et l'axe de la roue
- E est le module d'Young
- S est l'aire de la section droite de chaque rayon.

Dans un exemple de réalisation, en utilisant une jante, un moyeu et des rayons du commerce, on a obtenu les valeurs suivantes :

- h = 0,163
- k = 0,0224.

Cette fonction du troisième degré correspond à une grande sensibilité de la roue aux couples faibles ou très faibles, et à une bonne résistance de la roue à l'encontre des déformations au-delà d'un certain seuil de déformation d'environ 6° par exemple. La sensibilité aux faibles couples procure au dispositif l'avantage de fournir une mesure effective de ceux-ci. La réticence de la roue à dépasser un certain seuile de déformation est évidemment favorable pour la tenue mécanique et évite de plus les saturations dans la partie électronique du dispositif. L'écart angulaire entre les créneaux 41 successifs est choisi sensiblement plus grande que la déformation angulaire maximale susceptible de se rencontrer en pratique.

La tension mécanique des rayons peut être choisie assez librement, entre 100 et 400 Newton par exemple. Le calculateur comporte une entrée 60 pour y entrer les valeurs de $N_0$.

On va maintenant décrire le fonctionnement de la bicyclette, en y incluant la description du procédé selon l'invention :

Au repos, c'est-à-dire en l'absence de couple transmis, les rayons prennent une position radiale (lorsque la roue est vue axialement) sous l'effet de leur tension. La position angulaire relative du disque 21 et du cerceau 36 est telle que les perçages 22 et les créneaux 41 déclenchent simultanément la montée des signaux Ea et Eb respectivement de sorte que le signal S est constamment au niveau 0 et le calculateur indique pour C la valeur 0.

Lorsque la chaîne 7 transmet un couple de rotation à la roue 6, le moyeu 11 tend à tourner plus rapidement que la jante 13, tandis que les rayons s'inclinent par rapport à leur position initiale radiale. Le décalage angulaire entre moyeu et jante est détecté par le déphaseur 57 et l'intégrateur 58. Le calculateur détermine le couple d'après la formula (1).

Comme on l'a vu, pour détecter l'écart angulaire, on détecte le passage des repères 22 liés au moyeu 11, et des repères 41 liés à la jante 13, et on déduit l'écart angulaire d'après le retard des repères 41 par rapport aux repères 22.

De plus, on utilise la détection du passage des repères 22 pour mesurer en outre la vitesse de rotation V de la roue.

Comme on l'a vu, des grandeurs telles que la puissance P et l'énergie W liées à la vitesse V et au couple C sont également calculées.

On pourrait encore calculer des grandeurs liées uniquement au couple, par exemple le couple moyen, ou liées uniquement à la vitesse, comme par exemple la vitesse moyenne.

L'invention est applicable à d'autres roue, et à d'autres types de véhicules, notamment les cycles automoteurs.

## Revendications

1. Procédé pour mesurer le couple (C) transmis par la roue motrice (6) d'un cycle, ou une grandeur mécanique (P, W) fonction dudit couple, consistant à mesurer l'écart angulaire entre une partie menante et une partie menée de l'un des éléments rotatifs du cycle correspondant, faisant partie de ses éléments participant à sa propulsion, caractérisé en ce que l'on mesure la déformation angulaire engendrée dans la roue motrice (6) elle-même lors du roulement du cycle, par mesure de l'écart angulaire (A) existant de ce fait entre une région centrale (11) de cette roue motrice (6) et une région périphérique (13) de cette même roue, et ce en détectant le passage de deux séries distinctes de repères (22, 41) portés respectivement par la région centrale (11) de la roue motrice (6) et une région périphérique (13) de cette même roue, et on détermine le couple (C), ou la grandeur (P, W) qui lui est liée, en exploitant une règle de correspondance prédéterminée entre l'écart (A) détecté et le couple (C).

2. Procédé selon la revendication 1, caractérisé en ce qu'on utilise la détection du passage de certains au moins des repères (22) pour mesurer en outre la vitesse de rotation (V) de la roue (6) et/ou au moins une grandeur (P, W) qui lui est liée.

3. Dispositif pour la mise en œuvre du procédé selon l'une des revendications 1 ou 2, caractérisé en ce qu'il comprend des premiers moyens détecteurs (9) pour détecter la position angulaire d'une région centrale (11) de la roue motrice (6) autour de son axe (4), des seconds moyens détecteurs (12) pour détecter la position angulaire d'une région périphérique (13) de la roue motrice (6) autour de son axe (4), des moyens (57, 58) reliés aux premiers et seconds moyens détecteurs et conçus pour délivrer un signal représentatif de l'écart angulaire entre la région centrale (11) et la région périphérique (13) autour de l'axe (4) de la roue motrice (6), et des moyens (59) pour déterminer le couple transmis (C) ou la grandeur qui lui est liée par exploitation de la règle de correspondance entre l'écart angulaire (A) et le couple transmis (C).

4. Dispositif selon la revendication 5, caractérisé en ce que chacun des premiers (9) et seconds (12) moyens détecteurs comprend d'une part un élément (21, 36) destiné à être fixé à la roue motrice (6) et présentant au moins un repère (22, 41), et d'autre part un capteur (23, 40) fixable à un cadre (1) en position voisine d'une partie de trajet suivi par le repère (22, 41) lors de la rotation de la roue (6) et adapté à fournir un signal sensiblement binaire (Ea, Eb) dont le niveau est représentatif du passage du repère (22, 41) devant le capteur (23, 40).

5. Dispositif conforme à la revendication 4, caractérisé en ce que, à titre de repère, l'élément (21, 36) présente au moins un ajour (22, 41).

6. Dispositif conforme aux revendications 4 ou 5, caractérisé en ce que le capteur (23, 40) de chacun des premiers et seconds moyens détecteurs (9, 12) est du type photoélectrique.

7. Dispositif selon l'une des revendications 4 à 6, caractérisé en ce qu'il comprend en outre des moyens (61) pour déterminer d'après le signal sensiblement binaire (Ea) la vitesse (V) de rotation de la roue motrice (6) et/ou une grandeur liée à celle-ci.

8. Dispositif selon l'une des revendications 3 à 7, caractérisé en ce qu'il inclut la roue motrice (6) du cycle, dans laquelle la règle de correspondance donnant le couple transmis (C) en fonction de l'écart angulaire (A) est au moins du troisième degré.

9. Dispositif selon l'une des revendications 3 à 7, caractérisé en ce qu'il inclut la roue motrice (6) du cycle, laquelle est une roue à rayons fils (72) dont, vue axialement, les rayons fils (72) sont dirigés radialement.

## Patentansprüche

1. Verfahren zur Messung des vom Antriebsrad (6) eines Fahrrads übertragenen Drehmoments (C) oder einer von diesem Drehmoment abhängigen mechanischen Größe (P, W), bei dem der Drehwinkel zwischen einem antreibenden Teil und einem angetriebenen Teil eines der rotierenden Elemente des entsprechenden Fahrrades, die zu dessen Antriebselementen gehören, gemessen wird, dadurch gekennzeichnet, daß während des Rollens des Fahrrades die Verwindung des Antriebsrades (6) selbst gemessen wird, indem der durch die Verwindung erzeugte Drehwinkel (A) zwischen einem zentralen Bereich (11) des Antriebsrades (6) und einem äußeren Bereich desselben Rades gemessen wird, und indem das Vorbeilaufen zweier unterschiedlicher Folgen von Markierungen (22, 41) detektiert wird, welche jeweils an dem zentralen Bereich (11) des Antriebsrades (6) und an einem äußeren Bereich (13) desselben Rades angebracht sind, und daß das Drehmoment (C) oder die Größe (P, W), die von diesem abhängig ist, bestimmt wird, indem eine festgelegte Korrespondenzregel bezüglich des detektierten Winkels (A) und des Drehmoments (C) ausgewertet wird.

2. Vorrichtung nach Anspruch 1, dadurch gekennzeichnet, daß das Detektieren des Vorbeilaufens zumindest bestimmter Markierungen (22) dazu verwendet wird, zusätzlich die Rotationsgeschwindigkeit (V) des Rades (6) und/oder zumindest eine mit ihr verbundene Größe (P, W) zu messen.

3. Vorrichtung zur Durchführung des Verfahrens nach einem der Ansprüche 1 oder 2, dadurch gekennzeichnet, daß sie erste Detektionsmittel (9) aufweist, um die Winkelposition eines zentralen Bereiches (11) des Antriebsrades (6) um seine Achse (4) zu bestimmen, daß sie zweite Detektionsmittel (12) aufweist, um die Winkelposition eines äußeren Bereiches (13) des Antriebsrades (6) um seine Achse (4) zu bestimmen, daß sie Mittel (57, 58) aufweist, welche in Verbindung mit den ersten und zweiten Detektionsmitteln stehen und dafür vorgesehen sind, ein Signal zu liefern, das den Drehwinkel zwischen dem zentralen Bereich (11) und dem äußeren Bereich (13) um die Achse (4) des Antriebsrades (6) repräsentiert, und daß sie Elemente (59) aufweist, um das übertragene Drehmoment (C) oder die damit verbundene Größe durch Auswertung der Korrespondenzregel zwischen dem Drehwinkel (A) und dem übertragenen Drehmoment (C) zu bestimmen.

4. Vorrichtung nach Anspruch 3, dadurch gekennzeichnet, daß jedes der ersten (9) und zweiten (12) Detektionsmittel einerseits ein Element (21, 36) besitzt, das dafür bestimmt ist, am Antriebsrad (6) befestigt zu werden und mindestens eine Markierung (22, 41) aufweist, und andererseits einen Sensor (23, 40) besitzt, der an einem Rahmen (1) in einer Position fixierbar ist, die einem Teil der Bahn, welcher die Markierung (22, 41) während der Rotation des Rades (6) folgt, benachbart ist, und der in der Weise ausgebildet ist, daß er ein im wesentlichen binäres Signal (Ea, Eb) liefert, dessen Wert das Vorbeilaufen der Markierung (22, 41) vor dem Sensor (23, 40) repräsentiert.

5. Vorrichtung nach Anspruch 4, dadurch gekennzeichnet, daß das Element (21, 36) zur Bildung einer Markierung zumindest einen Durchbruch (22, 41) aufweist.

6. Vorrichtung nach den Ansprüchen 4 oder 5, dadurch gekennzeichnet, daß der Sensor (23, 40) jedes der ersten und zweiten Detektionsmittel (9, 12) als photoelektrischer Sensor ausgebildet ist.

7. Vorrichtung nach einem der Ansprüche 4 bis 6, dadurch gekennzeichnet, daß sie zusätzlich Elemente (61) aufweist, um aus dem im wesentlichen binären Signal (Ea) die Rotationsgeschwindigkeit (V) des Antriebsrades (6) und/oder eine mit ihr verbundene Größe zu bestimmen.

8. Vorrichtung nach einem der Ansprüche 3 bis 7, gekennzeichnet, dadurch daß sie das Antriebsrad (6) des Fahrrades beinhaltet, bei dem die Korrespondenzregel, welche das übertragene Drehmoment (C) als Funktion des Drehwinkels (A) liefert, zumindest dritten Grades ist.

9. Vorrichtung nach einem der Ansprüche 3 bis 7, gekennzeichnet, dadurch daß sie das Antriebsrad (6) des Fahrrades beinhaltet, welches als Speichenrad (72) ausgebildet ist, dessen Speichen (72), axial betrachtet, radial ausgerichtet sind.

## Claims

1. Process for measuring the torque (C) transmitted by the driving-wheel (6) of a cycle, or a mechanical value (P, W) function of said torque, consisting of measuring the angular variation between a driving part and a driven part of one of the rotating elements of the corresponding cycle, being part of the mechanism participating in its propulsion, characterized in that the angular deformation generated in the driving-wheel (6) itself when the cycle is running is measured by measuring the angular variation (A) existing as a result between a central region (11) of this driving-wheel (6) and a peripheral region (13) of this same wheel, and this by detecting the passage of two separate series of guiding-marks (22, 41) carried respectively by the central region (11) of the driving-wheel (6) and a peripheral region (13) of this same wheel, and the torque (C), or the value (P, W) which is linked to it, is determined by applying a predetermined rule of correspondence between the distance (A) detected and the torque (C).

2. Process according to Claim 1, characterized in that the detection of the passage of at least some of the guiding-marks (22) is utilized in order to measure the speed of rotation (V) of the wheel (6) and/or at least a value (P, W) which is linked to it.

3. Device for implementing the process according to one of Claims 1 or 2, characterized in that it includes first detecting means (9) for detecting the angular position of a central region (11) of the driving-wheel (6) around its axle (4), second detecting means (12) for detecting the angular position of a peripheral region (13) of the driving-wheel (6) around its axle (4), means (57, 58) linked to the first and second detecting means and designed to deliver a signal representative of the angular variation between the central region (11) and the peripheral region (13) around the axle (4) of the driving-wheel (6), and means (59) for determining the torque transmitted (C), or the value which is linked to it, by applying the ruel of correspondence between tha angular variation (A) and the torque transmitted (C).

4. Device according to Claim 5, characterized in that each of the first (9) and second (12) detecting means includes on the one hand an element (21, 36) intended to be fixed to the driving-wheel (6) and having at least one guiding-mark (22, 41), and on the other hand a pick-up (23, 40) which can be fixed to a frame (1) in a position adjacent to a part of the path followed by the guiding-mark (22, 41) on rotation of the wheel (6) and adapted to provide an obviously binary signal ($E\underline{a}$, $E\underline{b}$) of which the level is representative of the passage of the guiding-mark (22, 41) in front of the pick-up (23, 40).

5. Device according to Claim 4, characterized in that, as guiding-mark, the element (21, 36) has at least one hole (22, 41).

6. Device according to Claims 4 or 5, characterized in that the pick-up (23, 40) of each of the first and second detecting means (9, 12) is of the photoelectric type.

7. Device according to one of Claims 4 to 6, characterized in that it also includes means (61) for determining, according to the obviously binary signal ($E\underline{a}$), the speed of rotation V of the driving-wheel (6) and/or a value linked to this.

8. Device according to one of Claims 3 to 7, characterized in that it includes the driving-wheel (6) of the cycle, in which the rule of correspondence giving the torque transmitted (C) in terms of the angular variation (A) is at least of the third degree.

9. Device according to one of Claims 3 to 7, characterized in that it includes the driving-wheel (6) of the cycle which is a wheel with wire spoked (72), the wire spokes (72) of which, when viewed axially, are arranged in a radial direction.

FIG.1

EP 0 270 439 B1

FIG_2

FIG_3

FIG_4

FIG_5

EP 0 270 439 B1

$$C = hA^3 + kN_0A$$

FIG. 6